Europäisches Patentamt

European Patent Office

Office européen des brevets

(19)

(11)    **EP 1 624 302 A2**

(12)    **EUROPEAN PATENT APPLICATION**

(43) Date of publication:
    **08.02.2006 Bulletin 2006/06**

(51) Int Cl.:
    ***G01N 33/36*** (2006.01)

(21) Application number: **05019203.8**

(22) Date of filing: **08.01.2003**

(84) Designated Contracting States:
    **AT BE BG CH CY CZ DE DK EE ES FI FR GB GR
    HU IE IT LI LU MC NL PT SE SI SK TR**
    Designated Extension States:
    **AL LT LV MK RO**

(62) Document number(s) of the earlier application(s) in
    accordance with Art. 76 EPC:
    **03700021.3 / 1 581 807**

(71) Applicant: **Premier Evolvics PVT. Ltd.
    Coimbatore 641 005 (IN)**

(72) Inventors:
    • **Ramachandran, Shekaripuram, Narayanaswamy
      K.K. Pudur
      Coimbatore-641 038
      Tamil Nadu (IN)**

    • **Srinivasan, Varadarajan
      Coimbatore-641 045
      Tamil Nadu (IN)**
    • **Pavendhan, Appavu
      Coimbatore-641 015
      Tamil Nadu (IN)**

(74) Representative: **Frei Patent Attorneys
    Frei Patentanwaltsbüro
    Postfach 1771
    8032 Zürich (CH)**

Remarks:
    This application was filed on 05 - 09 - 2005 as a
    divisional application to the application mentioned
    under INID code 62.

(54)    **Measuring and testing continuous elongated textile material**

(57)    A device for determining optical characteristics
of a thread-like element comprises a non-monochromatic
light source (52) and a light frequency sensitive light de-
tector (55) being placed in a manner that the thread-like
element is illuminated by light emitted from the light
source and that a proportion of light emitted from the
thread-like element upon illumination by the light source
is directed to the detector. Signal processing means (56)
are communicatively coupled to the detector and are programmed in a manner that they can carry out the following
steps:

•    from a time dependent signal of the detector (55),
     evaluating a time dependent colour signal indicative
     of the colour of thread-like element,

•    from the time dependent colour signal, evaluating a
     time dependent colour deviation information indicative of deviations of the colour from the average colour of the thread-like element,

•    correlating said time dependent colour deviation information with a time dependent quantitative signal
     deviation information, and

•    determining, based on information whether the time dependent colour deviation information and the time
dependent total signal deviation are correlated, fault
information indicative of the nature of faults of the
thread-like element.

Fig.5

## Description

FIELD OF THE INVENTION

The invention is in the field of measuring or testing quality parameters of textile material. It particularly relates to methods, devices, and apparatus for testing elongated material.

BACKGROUND OF THE INVENTION

For the formation and the quality of fabric, the characteristic of yam used is critical. During the formation of fabric, the yarn used is subjected to varying tensions and speeds. As a result, yam exhibiting poor response to these conditions break and bring down the efficiency of the processes. The appearance of the fabric is also primarily determined by the variation present in the yarn. At the outset, the determination of yarn quality with respect to the mass variations, and diameter variations is inevitable.

All quality deviations identified at the final yam stage are not correctable; it is rather the preparatory processes which were responsible for the quality deviations that should be identified and corrected. For these reasons, the quality of yam is continuously monitored from the fibre stage to yam stage for various quality characteristics causing the deviations.

In the modem spinning environment, the immediate identification of the deviations of the materials during any process and the source is an important requirement. This is mainly because:

1. The machinery speeds employed with the modem machinery keep improving. As a result, even if there is a delay of a minute response, large quantity of poor quality material will be produced. An example of a modem draw frame runs at 1000 metres/ min.

2. At present, majority of the mills employ only one evenness tester for the measurement of mass properties. To satisfy condition 1, usage of more than one instrument may be required, since, in the present configuration of all available evenness testers, only one material can be tested at a time. This is because existing evenness testers comprise a capacitance measurement arrangement that works on a bridge configuration, wherein usually 4 slots are available to test, namely for testing sliver, roving, coarse spun yam and fine spun yarn. However, such a bridge configuration imposes the restriction that only one measurement can be done at a time using only one of these slots, and the other slots are required to form part of the measurement process. For this reason, it is not possible to test more than one material at a time. Further, the feeding mechanism of sliver, roving and yam are different in nature. Hence, these feeding arrangements need to be interchanged every time a different material type is to be tested. For these reasons, more than one instrument is required, along with more manpower to operate the same. The increasing operating manpower cost and investment on more than one instrument will put additional strain on the profit margins of the textile industry.

An important material parameter of spun yarn is the hairiness.

Spun yarns are produced using different spinning methodology. With the popular ring spinning methodology, the yarn is manufactured in ring frame machine by drawing out the roving, which is a bunch of fibres arranged in parallel and held together with a low twist, by inserting twist and by winding the yam on bobbins. The yam thus spun from the bunch of fibres has protruding fibres and looped fibres projecting from the core of the yam. These are called hairs, and the quantity and properties of the hairs of the yam are normally termed as yarn hairiness. The availability of hair along with the spun yarn is a common incidence with all types of spun yarns; with only the degree of hair formation varying with fibre properties, set of machinery parameters employed and different spinning methodology applied. Example of a recent Compact Spun methodology claims reduction in hairs compared to the conventional ring spinning methodology.

Yarn hairiness is a critical yarn parameter to the weaving and knitting processes with the increased level of hairiness resulting in breaks in warping and weaving, and high speed knitting processes. For example, the performance of an air jet spun yam during weaving is critically dependent on the level of hairiness. The yam hairiness is not always undesirable; certain applications prefer hairy yarn for better fabric cover and feel. Among the hairs available in a yarn arrangement, it is the longer length hairs, which critically influence the fabric finish like the dye absorption etc., than the short hairs. Thus, the measurement of yarn hairiness is important for optimising spinning process and produce yarn with desired quality.

Among various hairiness measurements - presently as many as 70 different measurement techniques have been suggested - there are only a few measurement techniques, which are popular. One of the measurement techniques is identified with Zweigle Hairiness Tester. The tester employs a light source, the light of which falls over the protruding hairs. The image of the protruding hairs is received by 12 phototransistors, which are closely spaced. The fibre length of each protruding hair is measured and expressed as hairs at certain class intervals.

Another invention, described in US Patent 5,875,419, by Lawson - Hemphill, Inc. claims a similar approach to the previous case with difference made in accounting the yam diameter to the hairiness measurement. Elongated textile material is moved passed a light source to create a shadow. The images are captured on a number of separate, close light sensing elements, each with an output level related to the amount of light shining on the element. The width of a number of closely spaced portions of the elongated material is determined, at two different selected sensing elements output levels, to distinguish the elongated material hairs from the core. In simple, the hairiness is expressed as a ratio of projected length to the elongated material diameter.

Another invention, disclosed in European Patent Application Publication EP-A-0'754'943, by Keisokki Kogyo Co., uses a device with which it is possible to detect data for the core part and the hairy part individually. The elongated material-measuring device is provided with means for forming a Fourier transformation pattern, including a elongated material core pattern and a surface projection pattern, on a spectrum plane, by performing a Fourier transformation on diffracted laser light, which was intersected and passed through travelling elongated material. The hairs are not individually accounted and only the total quantum of hairs per unit length is measured with this invention.

Yet another invention, described in US patent 4,948,260, by Zellweger Uster Limited, measures yarn hairiness similar to the mentioned European Patent Application Publication EP-A 0'754'943, however uses a device comprising a light transmitter for illuminating the test body from one side and a receiver for receiving and evaluating light reflected by the surface of the test body depending on the structure of the surface, a screen means having an optical system with a central diaphragm, and an absorber screen placed between the transmitter and the receiver for screening the direct light from the illuminated side of the test body wherein the receiver detects only light which is reflected from one side of the test body.

Both in Zweigle Hairiness tester and in Lawson - Hemphill system and method, the length of hair is measured either in relation with yam diameter or without it. However, since the hairs are not always available in perpendicular to the yarn axis in a straight line, (normally hairs protrude in all directions in different shapes including bent, inclined and looped), the length of hairs measured are not the true length and also no mention is available for the correction of this fact in either of these methods.

## SUMMARY OF THE INVENTION

It is an objective of this invention to provide a method and an apparatus as well as a device which contribute to improved elongated textile material testing, especially in view of the above mentioned requirements. "Elongated (textile) material" or "thread-like element", in the following, may relate to any form of intermediate or final product in a textile manufacturing process, such as, for example, to sliver, roving or yarn.

For these discussed requirements, an apparatus is proposed which can test more than one test specimen being a thread-like element (in the broadest sense of the word). The apparatus may test thread-like elements of different natures including yarn, sliver and roving, simultaneously employing a different set of test conditions for the estimation of the quality characteristics such as, for example, hairiness properties, fault including neps, mass properties and linear density (count). The proposed apparatus can preferably provide the possibility to be managed with the same number of manpower employed at present for state of the art evenness testers.

It is a further objective of this invention to provide an apparatus to measure the various physical properties of more than one continuous elongated material simultaneously.

The apparatus thus contains at least two arrangements, each test arrangement comprising at least a module for measuring the linear density of a test specimen and a mass measurement module. It comprises a signal processor and/or controller for evaluating signals from the modules of both test arrangements. At least one of the test arrangements may optionally further comprise a module for measuring the hairiness properties, and/or a module for measuring and classifying certain type of faults in terms of its optical characteristics.

For example, the first test arrangement, optionally comprising the hairiness properties module and/or the module for measuring and classifying certain types of faults, may comprise feeding means for feeding yarn, whereas the second test arrangement may comprise feeding means for feeding sliver and/or roving - thus making a simultaneous measurement of yam properties and sliver and/or roving properties in one apparatus possible without the need to often interchange the feeding means.

Based on the requirement of testing more than one test specimen and the set of physical properties, a number of separate test arrangements for each of the test specimen can be arranged. Each of these arrangements will contain modules intended for testing the required physical properties. Each of these test arrangement may be supported with automatic test specimen feeding means for placing the test specimen on the chosen test specimen path for the uniform test conditions. These test arrangements can also be manually fed.

It is a further objective of this invention to provide a mechanism to measure the linear density of the test specimen.

In one preferred apparatus, the test specimen travels on its defined path. The test specimen to the specified length is cut and collected by a collecting device, which travels on a perpendicular plane to the test specimen. The collected test specimen is then placed on a weighing scale.

In another preferred apparatus, the test specimen travels in a linear path and after the specified length meant for linear density measurement crosses a reference point, the test specimen is cut and diverted of its path. Meanwhile, the cut material, which travelled in the linear path, is deposited on a weighing scale.

It is a further objective of this invention to provide a mechanism to measure the optical properties of the test specimen. In the preferred arrangement, the test specimen, when it crosses the sensor assembly, intersects the beam path of a polychromatic (non-monochromatic) light beam. The reflected light from the test specimen is collected for example as three chromatic components (red, green and blue) by a detector or a plurality of detectors. The impurities available in the test specimen are classified according to the response made with respect to chromatic components.

"light" in the context of this entire application, refers to electromagnetic radiation in the general sense including infrared, visible and ultraviolet radiation.

As a collective advantage of these inventions, measurements of quality characteristics like mass variation, hairiness, optical characteristics and linear density can be done on a single test specimen. This results in increasing number of tests per unit time as well as saving of test specimen length.

It is also an objective of this invention to provide a hairiness tester module and a hairiness tester device as well as a method to measure the length of hairs as available in the elongated material and as presented in the measuring arrangement and also to measure the degree of non-linearity of hairs.

Among short hairs and long hairs, long hairs are particularly a disturbing factor in the fabric formation processes including weaving and finishing processes. Since the long hairs have a higher tendency, because of the linear density, to drape, loop and be protrude in any direction in reference to the elongated material axis in conventional measurements, hairs are assumed to be available in perpendicular to the thread-like element axis, the length of hair measured as available in the thread-like element is actually not the full length. A non-linearity factor accounting for the properties of loop, drape, among others would help predicting the behaviour of thread-like element such as a textile yam in fabric processing much better from the conventional methods of measurement outputs.

It is therefore a further objective of this invention to provide a device and a method for measuring the hairiness and accounting for the non-linearity of hairs. Preferably, the invention should provide a method and a device that measures the relation between the diameter of hairs and the scattered/reflected light of the same hairs using the proposed devices.

According to the invention, this is accomplished by an optical method which evaluates two signals, a total hair length signal indicative of the total, real length of hairs existing in a measuring zone and a total hair protrusion signal indicative of how far hairs protrude in the measuring zone, for example given by how far the shadow reaches out from the thread-like element. A non-linearity factor may be calculated based on a quotient of the total hair protrusion signal and the total hair length signal.

Preferably, the apparatus for carrying out this method comprises a light source having coherent linear light and therefore giving a clear, distinct shadow picture, and an array of detectors, for example including CCD elements, to measure position sensitive signals for evaluating the non - linearity of hairs in the thread-like element.

The light source may emit light in the visible and invisible spectral range of light, and the device comprises a detector having wide spectral response to detect the reflected/refracted/scattered light of the hairs in the thread-like element.

The above preferred method brings about a specific advantage when used with thread-like elements being cotton yam. Cotton yarn in its natural colour is desired. Primary among factors influencing the light scattering of cotton yarn hairs in its natural colour is circularity of hairs and the thickness. The colour variation of cotton fibre hairs is expected to influence less in comparison with the variation in circularity of cotton fibres. The circularity of a cotton fibre is primarily determined by the degree of maturity with a matured fibre showing a higher cell wall thickness compared to an immature fibre and dead fibre. The matured fibre reflects more light in comparison with a dead fibre. By normalising the quantum of scattered light to the hair thickness, determined using a said detector as shadow, it is possible to select a cotton yam between choices for a preferred end use application like mercerising and dyeing and also to optimise use of raw material and process. In one of the preferred end use application-dyeing, lack of dyeing uniformity in cotton products has been a long standing problem for textile processors. Dyeing problem with cotton products are primarily associated with maturity levels of fibres.

BRIEF DESCRIPTION OF THE DRAWINGS

In the following, preferred embodiments of this invention are described in further detail with reference to drawings. In the drawings, the Figures show the following:

Figs. 1A and 1B a 3D view and a side view, respectively, of an apparatus according to the invention,

Fig. 2 a scheme of a module for the linear density measurement,

Figs 2A through 2D a sequence showing the function of the module of Fig. 2,

Fig. 3 a scheme of another embodiment for the linear density measurement,

Figs 3A through 3C a sequence showing the function of the module of Fig. 3,

Fig. 4 a scheme of a module for the measurement of optical characteristics,

Fig. 5 signals measured with the module of Fig. 4 (schematically),

Fig. 6 an overall block diagram of the apparatus of Fig. 1A and Fig. 1B,

Fig. 7 a block diagram of a capacitance mass sensor module,

Figs. 8A through 8C different configurations of hairs protruding from textile spun yarn 8 (schematically),

Fig. 9 a side view (top) and a front view (bottom) of a first embodiment of a hairiness tester,

Fig. 10 a second embodiment of a hairiness tester,

Figs. 11 through 14 the response of an array of detectors in different situations (schematically),

Fig. 15 a third embodiment of a hairiness tester,

Fig. 16 an arrangement of hairs in a measuring zone,

Figs. 17A through 17C schemes showing the relation between fibre properties and measurement signals.

DESCRIPTION OF THE PREFERRED EMBODIMENTS

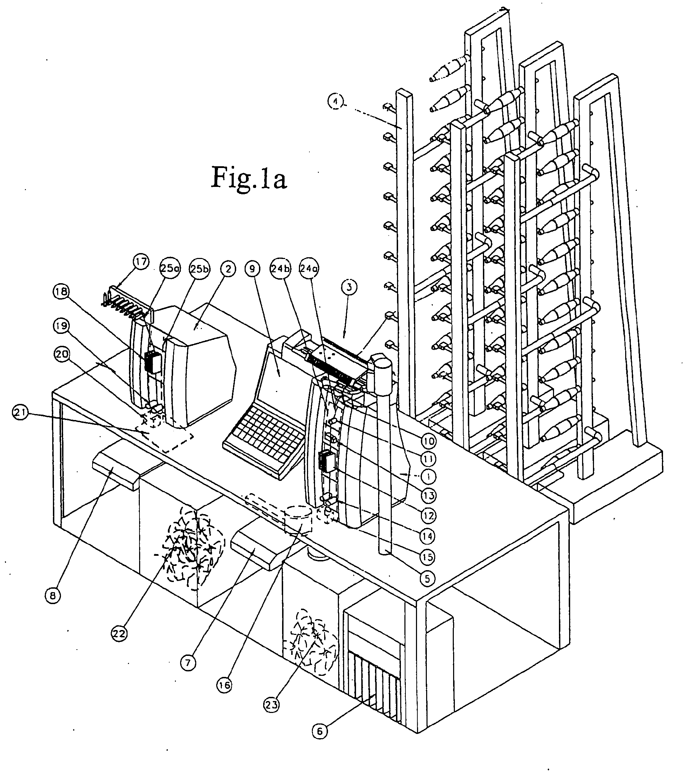

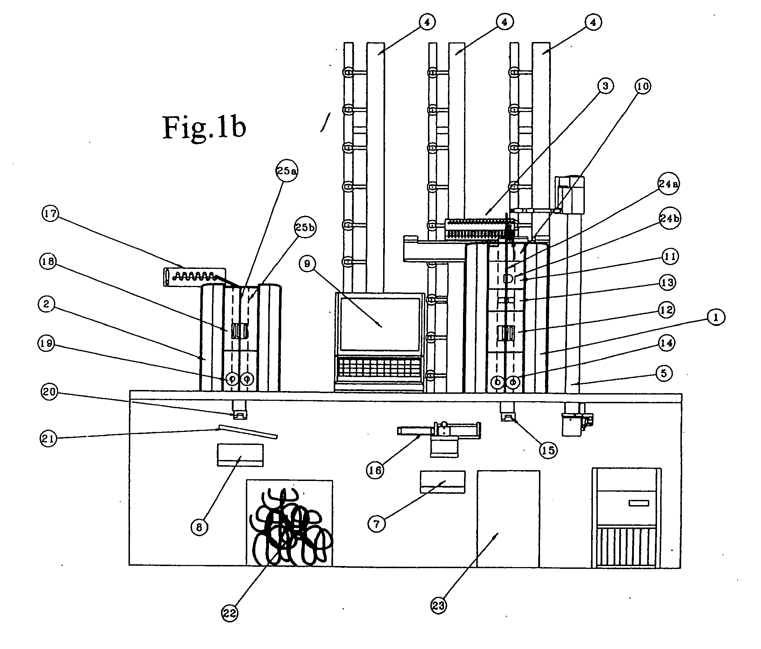

**Figures 1A and 1B** show an apparatus of a preferred embodiment for testing two test specimens simultaneously. Though the shown embodiment refers to testing two test specimens at a time, it is possible to integrate more than two test arrangements into one apparatus.

The embodiment contains a first test arrangement 1 for measuring a first test specimen 24a and a second test arrangement 2 for measuring another test specimen 25a. In the first test arrangement, the test specimen 24a kept in the creel 4 is drawn through a feeding mechanism 3 and laid in the test path 24b by a laying arm 5.

The second test arrangement 2 may, as an example, be used for testing sliver or roving. Often, the linear density and its variation of the spun yarn are influenced by the linear density of sliver and roving. This is because the expected linear density of sliver and roving may be altered in the process by variation present in the material, especially the long term variation introduced by machine especially its critical components.

The first test arrangement 1, according to this example, may be used for testing the spun yarn.

As an alternative, both, the first and the second test arrangement may be used for testing spun yam. As another alternative, the first and the second test arrangement may be used for testing sliver/roving. Then (or also in the other cases), the first test arrangement may comprise less then the totality of the modules that are described further below.

The creel 4 is designed to accommodate the test specimen packages; the packages are placed over a peg and drawn to the feeding mechanism through appropriate tensioning devices. The feeding mechanism 3 holds the starting end of the test specimens in an array of holders. During the start of every test, the laying arm 5 picks up a test specimen 24a from the holders in sequence and places on the test path 24b.

Along the test path in the first test arrangement 1, the modules are stacked one above the other. In Figures 1A and 1B, the following modules are present: A hairiness sensing module 10 meant for determination of the hairiness properties, a mass (capacitance) sensor 12 meant for the estimation of mass properties, an optical sensor 13 for the estimation and classification of impurities. These modules are stacked in a vertical plane. A tensioning device 11 is placed in the test specimen path 24b to give a pre- determined tension for the uniform test conditions between tests. A pair of rollers 14 -together with the arm 5 serving as feeding means - is placed downstream of the arrangement to draw the test specimen at a pre-selected withdrawal speed. Also this pair of rollers 14 guides the test specimen to the next possible measuring module, in this case a module for the measurement of linear density of the test specimen 24a. The linear density measuring module comprises of a cutter 15, a collection container 16 and a weighing scale 7. A waste collection box 23 is placed at the bottom of the test arrangement to collect the test specimen falling directly as well as the portion of the test specimen subjected to the linear density measurement.

The second test arrangement 2 in the example shown is similar to the first test arrangement 1. Here, the second arrangement does not have to be a complete arrangement, i.e. it can comprise only some modules of a full arrangement such as the first arrangement. In a preferred embodiment, in the second test arrangement 2, a capacitive sensor 18 is placed for the estimation of mass variation of the second test specimen 25a - for example a sliver or roving. The test specimen is guided to the test path 25b by feeding means 17. The test specimen travels in a linear path guided by a pair of conveyor rollers 19. In alternative embodiments, the path may be non-linear and for example contain deviations. The pair of conveyor rollers 19 guides the test specimen to the next possible measuring module, in this case a module for the measurement of linear density of the test specimen 25a. The linear density measuring module comprises of a cutter 20, a diverting plate 21 and a weighing scale 8 .A waste collection box 22 is placed at the bottom of the test arrangement to collect the test specimen falling directly as well as portion of the test specimen subjected to the linear density measurement.

The two test arrangements comprise common processing elements, for example common signal processor and controller means 6, which may be connected with a PC having a user interface. This fact may for example be used for correlated testing. If the second test arrangement tests sliver or roving, and the first test arrangement tests yam spun from such product, the data evaluating means may be provided with information about the spinning process in a manner that a certain signal of the second test arrangement may be correlated with a later signal of the first test arrangement, the later signal referring to a yam section of which the tested sliver or roving section is part.

Also, since the two test arrangements 1, 2 operate simultaneously and test two test specimens 24a, 25a, the total number of tests conducted per unit time is more in comparison to conventionally available evenness testers. Since multiple quality characteristics are estimated for the same test specimen length, a saving of test specimen is also envisaged. Further, by having an integrated apparatus containing two test arrangements, the required space is reduced compared to a double test apparatus. Also the time required for an operator operating two apparatus having two evaluation units is higher then the time required for operating an apparatus according to the invention.

Next, a device for linear density measurement is described in further detail. Such a device may but does not have to serve as a module in an apparatus as described with reference to Figs. 1A and 1B.

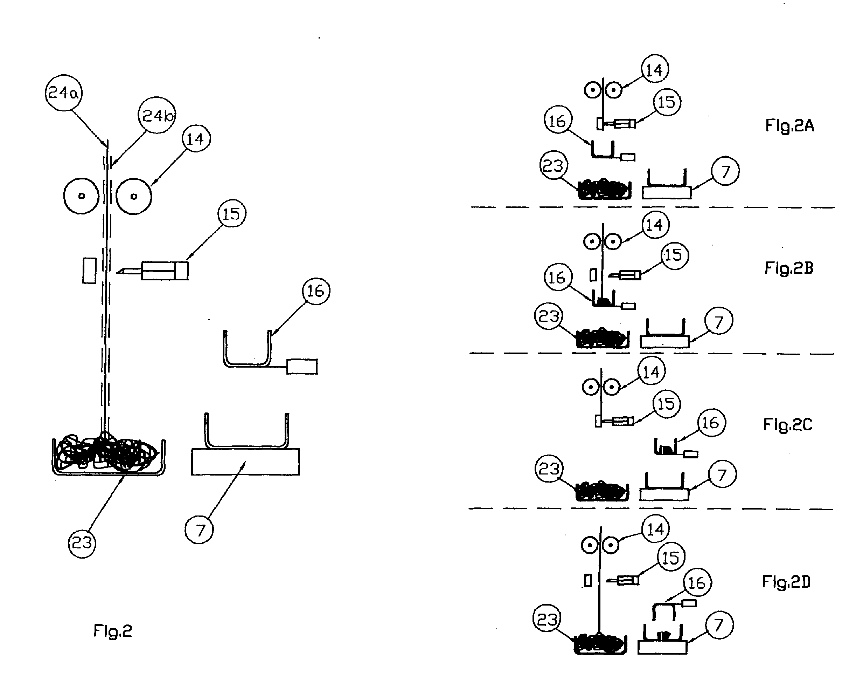

**Figure 2** accordingly shows a module for the measurement of linear density of a test specimen.

The measurement of linear density, in an apparatus according to the invention, is done simultaneously to that of mass, hairiness, optical characteristics, etc for the specimen under the test.

The arrangement comprises of a conveyor roller 14, which is part of a test arrangement 1 explained in Figure 1. Underneath the conveyor roller, on the test specimen running path 24b, a cutting arrangement 15 and the waste collection box 23 are arranged one below the other. A collection container 16 is positioned away from the test path. A weighing scale 7 is positioned near the waste collection box.

The sequence of operation preferred for the measurement is explained with reference to **Figures 2A - 2D**.

In Figure 2A, after the start of a measurement, material having a pre-defined length is collected directly in the waste box 23. Then the cutting arrangement 15 executes a cut on the running specimen 24a.

In Figure 2B, the collection container 16 comes into the specimen path 24b and starts collecting the running test specimen. The collection container, for this purpose, is provided with an appropriate transport mechanism, such as a hydraulics, pneumatics, electrical steppers, etc.

In Figure 2C, after the pre-defined test length of the test specimen is reached, the cutting arrangement 15 again initiates a cut on the running test specimen. Simultaneously, the collecting device 16 retreats to its original position with the cut test specimen.

In Figure 2D, the collecting device transfers the collected test specimen for measurement onto a weighing scale 7.

The determined weight is transferred to the controller 6 of Figure 1, where with a pre- defined formula, the linear density of the test specimen is evaluated by relating the weight and the length of the test specimen meant for the linear density measurement.

After the weighing, the test specimen is disposed to the waste collection box 23.

This sequence will repeat for each new test specimen as well as within the test specimen as desired.

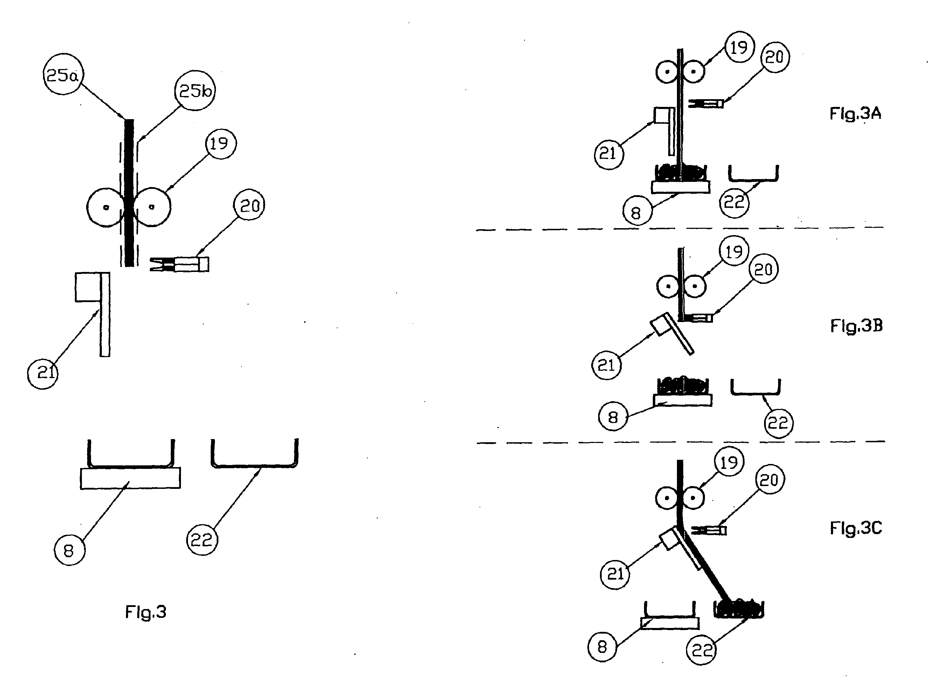

**Figure 3** shows another preferred embodiment for the measurement of linear density of a test specimen.

The arrangement comprises a conveyor roller 19, which is part of a test arrangement 2 explained in Figure 1. Underneath the conveyor roller, on the test specimen-running path 25b, a cutting arrangement 20 a diverting plate 21 and a weighing scale 8 are arranged one below the other. A waste collection box 22 is positioned away from the test path.

The sequence of operation preferred for the measurement is explained using the **Figures 3A-3C**.

In Figure 3A, after the start of a measurement, material equivalent to a preferred length for the linear density measurement is collected directly on the weighing scale 8.

In Figure 3B, the cutting arrangement 20 initiates a cut on the running test specimen 25a. Simultaneously, the diversion plate 21 comes into the specimen path 25b.

In Figure 3C, the test specimen is diverted by the diverting plate 21 to the waste collection box 22.

The determined weight from the weighing scale 8 is transmitted to the controller 6 of Figure 1, where with a pre- defined formula, the linear density of the test evaluated is defined by relating the weight and the length of the test specimen meant for the linear density measurement.

This sequence will repeat for each new test specimen, or it may be repeated for the same test specimen if desired.

Next, a device for evaluation of optical characteristics is described in further detail Such a device may (but does not have to) serve as a module in an apparatus as described with reference to Figs. 1A and 1B.

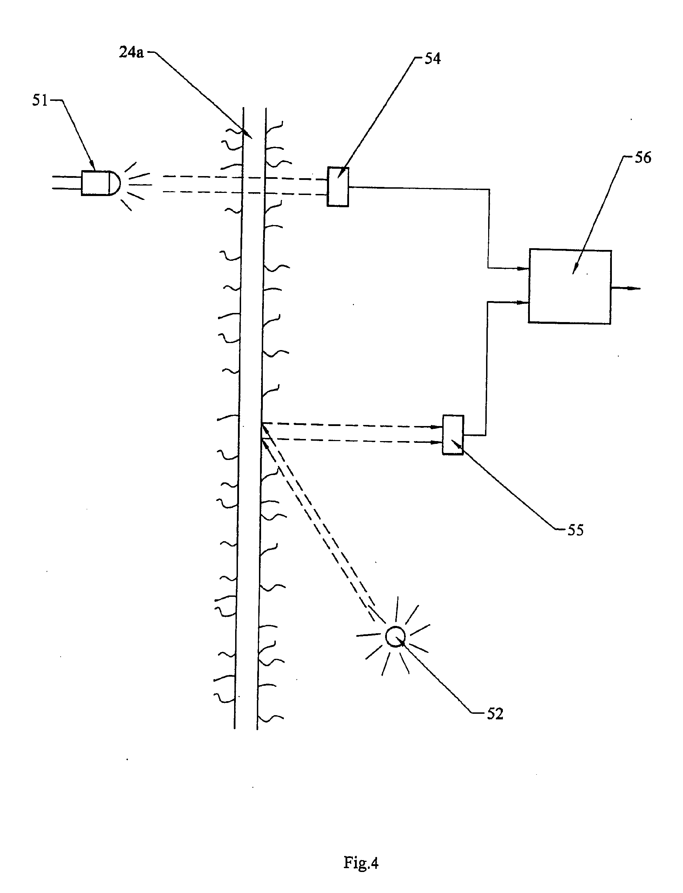

A preferred embodiment for the module for measurement of optical characteristics of the test specimen is shown in **Figure 4**. The module is denoted by 13 in Figure 1. The embodiment comprises a first light source 51, emitting electromagnetic radiation in a visible and / or invisible spectral range and a second light source 52, preferably a polychromatic light source, a first detector 54, preferably for detecting full spectral ranges, a RGB detector 55 and a signal processor 56. The test specimen 24a, the same as referred to in Fig.1, preferably the textile spun yarn for which the optical characteristics is to be estimated is positioned to receive the light from both light sources 51, 52.

When the test specimen 24a is moved between the first light source 51 and the detector 54, the core body of test specimen 53 is blocked depending on the thickness of the test specimen and the detector 54 receives light signal proportional to the thickness of the test specimen. The signal received by the detector is passed on to the signal processor 56.

The test specimen 24a on its further downward movement receives the light from the second light source 52. The test specimen reflects the light falling over and the RGB detector 55, which contains detectors separately for receiving Red, Green and Blue, receives the respective signal from the total reflected light. The signal received by the detector is passed on to the signal processor 56.

The signals provided by the detectors may be evaluated as described in the following.

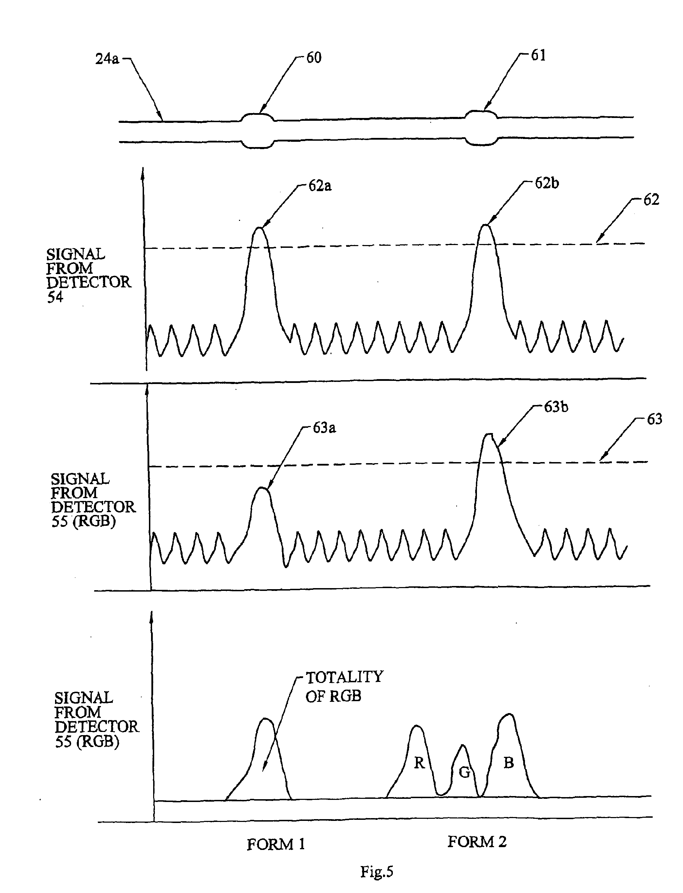

The determination of optical characteristic, preferably a fault, of the test specimen, preferably a textile spun yam, is explained with reference to **Figure 5**.

The test specimen 24a along its length contains two faults 60 and 61. The colour of the first fault 60 is similar to the test specimen colour and the colour of the second fault 61 is different from the test specimen colour.

The first fault 60 produces a signal, collected by the detector 54, of size 62a, which crosses the fault threshold 62 to be classified as a fault. Similarly, second fault 61 produces a signal 62b.

The first fault 60 produces an equivalent RGB signal, received from the detector 55, of size 63a, which falls below the set threshold for detecting the nature of the fault. In this case, since the colour of the fault 60 is similar to the nature of the test specimen, this fault is classified as Fault Type 1, which may be called 'Process Faults' .

The second fault 61 produces an equivalent RGB signal, received from the detector 55, of size 63 b, which crosses the set threshold 63. As a result, the fault 61 is classified as Fault Type 2, which may be called 'Raw material fault', which is predominantly caused by seed coat, seed, portions of leaf, bark, stem in the case of a cotton spun yarn.

The RGB signals are analysed in two forms as can be seen in the bottom panel of Fig. 5. In Form 1, the RGB signals are considered in totality using some hardware arrangement/reference algorithms. As an alternate, as described in Form 2, the individual responses of R, G and B detectors can be made use of using some hardware arrangement/reference algorithms.

The above described embodiments of the module for measuring optical characteristics (Fig. 4) and the according methodology for the determination of optical characteristics, preferably a fault of the test specimen, (Fig. 5) can also be employed in an on-line textile environment.

A preferred usage can be in yam winders wherein the yam is converted from a smaller package to a larger package, and in the process of doing so the specific nature of defects is monitored and/or removed, using an integrated arrangement such as one comprising yarn clearers.

The embodiment described with reference to Figs. 4 and 5 could be advantageously used as a yarn clearing device with the shadow inputs from the detector 54 used for the fault classification like the classification into "Nep", "Thick", "Thin" and the color signal inputs from the RGB detector 55 used for the selective clearing of yam faults such as Raw material faults and process faults.

Alternatively, the described embodiments can be arranged to work along with a capacitance type of yarn clearer.

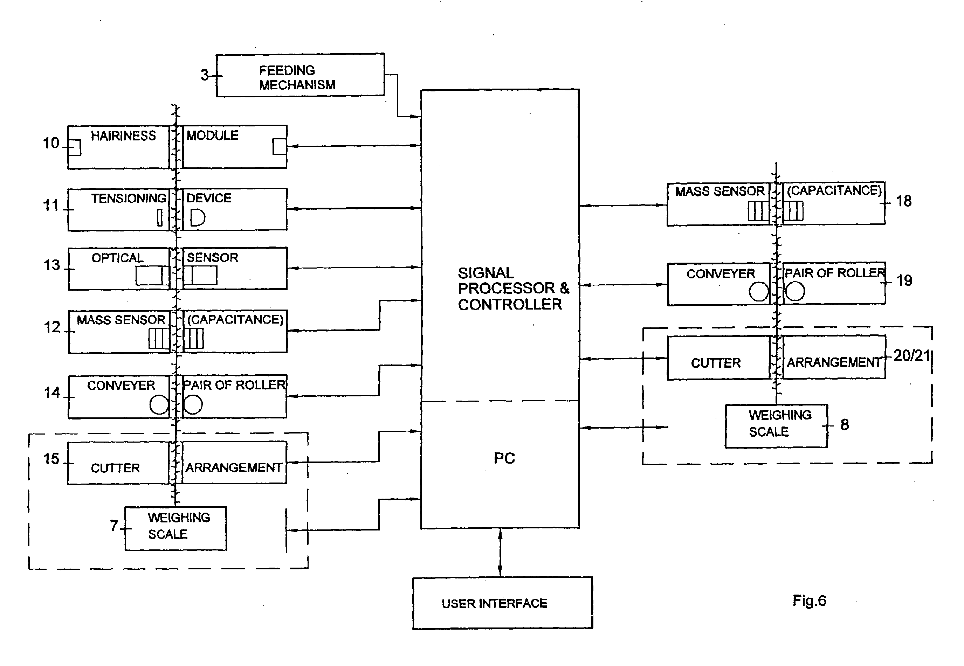

**Figure 6** provides a block diagram of the information flow in the apparatus of Figs. 1A and 1B. As can be seen from the diagram, a Personal Computer (PC) in control of a Signal Processor & Controller which, actually, may be incorporated in the (PC) controls and co-ordinates the feeding mechanism, the tensioning device and the respective modules of both arrangements (depicted, in the figure, on the left-hand side and on the right-hand side of the signal processor & Controller, respectively). The PC also controls the linear density measurements and is responsible for the evaluation.

It goes without saying that the PC does not have to be a personal computer in the narrow sense of the word but may be any computer controlling means provided with an appropriate user interface.

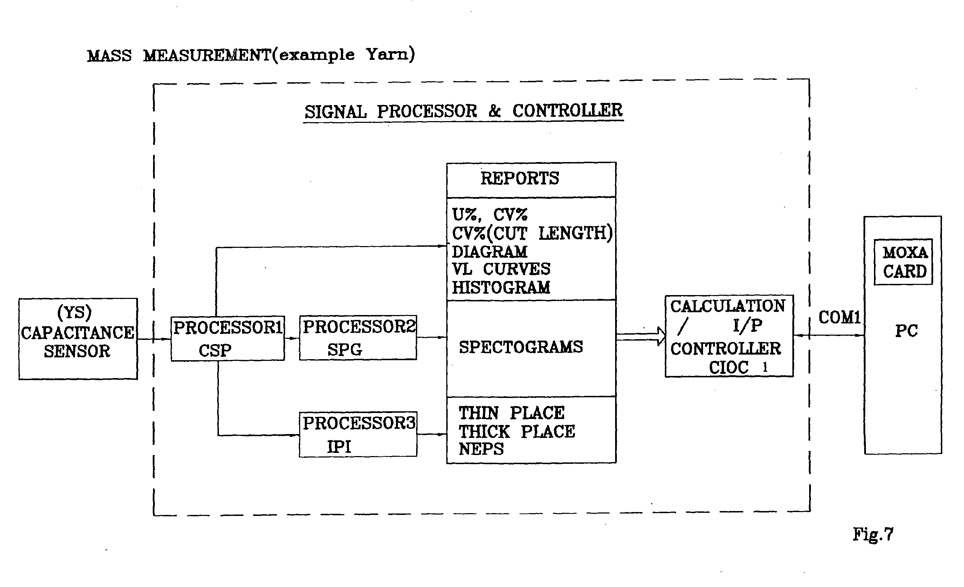

**Figure 7** shows a block diagram of the mass capacitance sensor. The different steps of the evaluation are depicted from left to right in the Figure. The steps are carried out by processors denominated by numbers 1-3 and by "calculation/ I/P Controller". The Signal Processor & Controller unit containing these processors communicates with the PC via an interface ("Com1" in the Figure).

Next, properties of an embodiment of a hairiness testing device and an according method are described in more detail. The hairiness testing device is a hairiness testing module in the above described apparatus or may - comprising appropriate feeding means - stand on its own or is a hairiness testing module in an other apparatus.

**Figure 8A** shows individual hairs 102, 103, 104 protruding from elongated material 101, preferably a textile spun yarn. Among the various possibilities of hairs arrangement, for the explanation of the methodology, three common arrangements of the hairs are preferred.

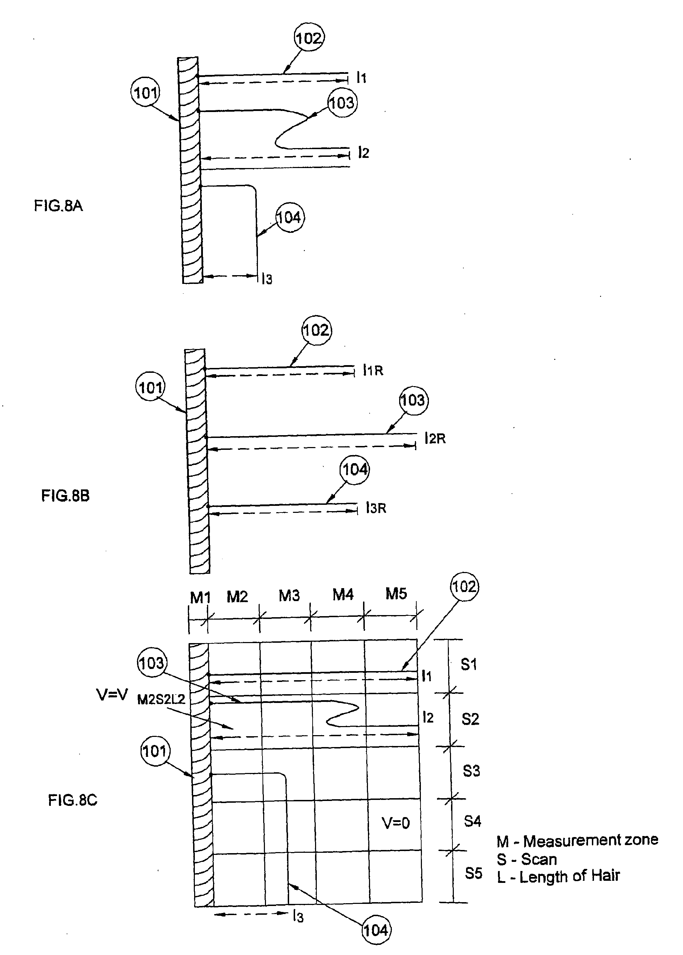

An elongated material having a core body 101, a straight hair 102 arranged in perpendicular to the yarn axis, a hair in wavy arrangement 103 and a draped hair 104 are shown in the Figure. The edges of these hairs are at a distance of $l_1$, $l_2$, and $l_3$ respectively from the surface of the core body 101. The lengths $l_1$, $l_2$, and $l_3$ are the length of hairs as available in the elongated material and as presented in the measuring arrangement. However, from **Figure 8B,** it can be seen that the actual length of the hairs are $l_{1R}$, $l_{2R}$, and $l_{3R}$ and are equal or different from $l_1$, $l_2$, and $l_3$.

Let the total length of hairs as available in the elongated material and as presented in the measuring arrangement be declared as $\Sigma L$ and calculated by adding $1_1$, $1_2$, and $1_3$. (Figure 8A).

Let the total actual length of hairs be declared as $\Sigma LR$ and calculated by adding $1_{1R}$, $l_{2R}$, and $1_{3R}$. (Figure 8B).

The non- linearity factor, which explains the degree to which the fibres are away from an ideal straight-line situation, is estimated by relating $\Sigma L$ and $\Sigma LR$. One of the preferred methods of non- linearity factor can be derived from the following equation

$$( \Sigma LR / \Sigma L * 100) - 100 \ldots (1)$$

The non - linearity factor in the preferred method is estimated as follows:

From **Figure 8C**, it can be seen that the core body 101 and the individual hairs are projected over measurement length zones of M1 and M2 onwards. The measurement length zone M1 is defined by the dimension of the core body 101 and zones M2 and onwards are equally spaced. They cover the region of the protruding hairs.

If the average individual signal value of a hair in a measurement zone (corresponding to a detector) estimated from initial runs and calibrations be V for a scan, then for example, in measurement zone M2, the 3 hairs 102, 103, 104 will return signal values equivalent to $V_{s1m211}$, $V_{s2m212}$ and $V_{s3m213}$ whereas a value of Zero (0) will be returned if no hair is present.

The individual signal value $V_{s1m211}$ can best be explained with the following nomenclatures.

"s" for the scanning zone, "m" for the measurement zone and "l" for the length of the hair.

As can be seen from Figure 8C, straight hair 102 protrudes to Measurement Zone M5, the wavy hair 103 also to Measurement Zone M5 and the drape hair only to Measurement Zone M3.

For the discussion, it is assumed that each measuring zone size ($M_1$) is 1 mm. Hence straight hair 102 is of length 4 mm, the wavy hair 103 is 4 mm and the drape hair 104 is 2 mm. The total length of the hairs ($\Sigma L$) as available in the elongated material and as presented in the measuring arrangement works out to 10 mm (4mm + 4mm+ 2mm).

$$\Sigma L = l_1 + l_2 + l_3 \ (2)$$

It can also be seen from Figure 8C that the 3 hairs occupy a total of 5 scans.

The total signal value of straight hair corresponds to

$$V_{s1m2l1} + V_{s1m3l1} + V_{s1m4l1} + V_{s1m5l1} \quad (3)$$

The total signal value of wavy hair corresponds to

$$V_{s2m2l2} + V_{s2m3l2} + V_{s2m4l2} + V_{s2m5l2}. \quad (4)$$

The total signal value of drape hair corresponds to

$$V_{s3m2l3} + V_{s3m3l3} + V_{s4m3l3} + V_{s5m3l3}. \quad \ldots (5)$$

It will be of importance to note that the signal value $V_{s2m4l2}$ will be higher than the adjacent values $V_{s2m3l2}$ and $V_{s2m5l2}$ by the proportion of the exposed length.

The total actual length of hairs is then estimated by adding total signal values of all 3 hairs from equations (3), (4) and (5) and dividing the total signal value by average individual signal value of a hair in a measurement zone per scan, V. The calculated value is multiplied by the measuring zone size ($M_1$).

$$\Sigma \, LR = \{((Eqn\ 3) + (Eqn\ 4) + (Eqn\ 5)) / V\} * M_l \quad (6)$$

The equation (6), for the discussion, applied on the configuration of Figure 8C will return a length of 4mm for straight hair; 6mm for wavy hair and 4 mm for drape hair. Thus giving a value of 14 mm for $\Sigma\, LR$.

Applying Equation (1), the non - linearity factor is estimated to be

$$(14 / 10 * 100) - 100 = 40.$$

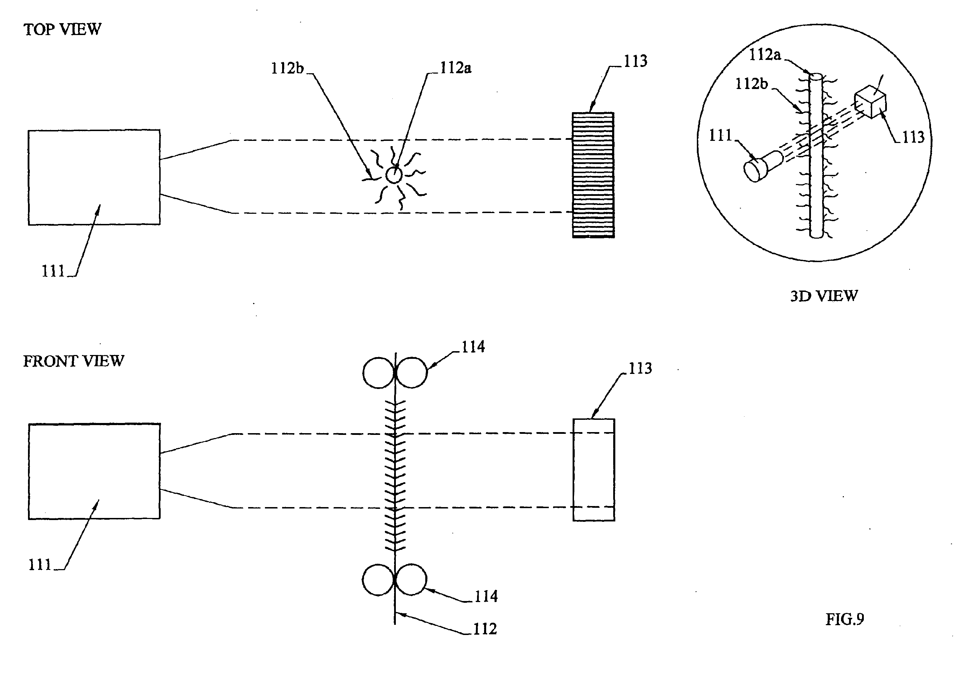

A preferred embodiment for measuring the total hair length and non-linearity factor as shown above can be found in **Figure 9**. The embodiment comprises a light source 111, preferably for producing a coherent linear beam, and an array of detectors 113 including CCD elements displaced at a pre-defined positions. The elongated material 112, preferably the textile spun yarn for which the yarn hairiness is to be estimated is positioned to run between the light source 111 and the detectors 113 using pairs of roller 114 running at a constant pre -selected speed. The detectors that are positioned at a distance from the light source receive the coherent linear light beam coming out of the source.

When an elongated material 112 is moved between the light source and the detectors, the core body 112a and the projected hairs 112b of the elongated material block the light passing through.

The detectors then receive only the light that have escaped through and travels in the desired path. The quantum of the light thus received by these individual detectors is in proportion to the thickness of the core body and the protruding hairs.

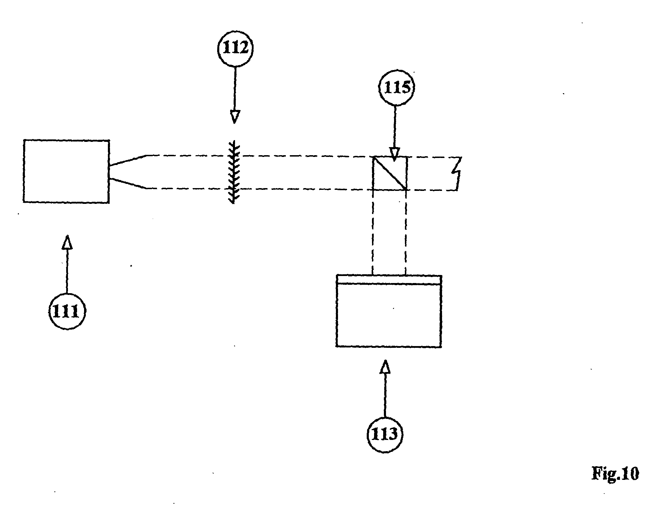

Another arrangement to this preferred embodiment employs a beam splitter 115 in between the light source 111 and the Detector 113 as shown in **Figure 10**. The beam splitter receives light from the source and splits the light into two different axes without deforming the shape of the beam. In Figure 8, the detector is positioned at an angle, preferably at 90 degree to the light source.

Signal Analysis:

It has been found that the average distance between two neighbouring hairs is a few millimetres in most cases, thus, considerably more than a diameter of a measurement zone, of for example around 1 mm. Therefore, it may safely be assumed that usually not more than one hair is present in one zone.

The detectors and the means for processing and evaluating the detected signals measure, as a function of time, a total, quantitative shadow signal being the sum of the signals V of all detectors except in the shadow zone of the core body 112a as explained above. This value is indicative of the total real length of the hairs $\Sigma\, LR$. Further, a time dependent protrusion signal is measured, indicative of the total apparent length $\Sigma\, L$ (if desired, the values can be integrated over some time in order to give total values representative of a certain yam length, this especially being desirable if the detectors are small). The time-dependent protrusion signal may be evaluated in the following manner: A threshold signal level per measurement zone is determined, which is exceeded in each measurement zone where a hair is present.

The protrusion signal is defined to be the distance from the outer edge of the yam diameter to the outermost measuring zone for which a signal exceeding the threshold is detected. Depending on calibration, the yarn diameter (divided by half) may be deducted from the protrusion signal; in the case of a high resolution, for an (optional) measurement of the yam diameter, the same method may be applied but with a much higher threshold value essentially indicative of regions which are entirely blocked from receiving sufficient light. From these signals, a non-linearity factor may be evaluated as explained above.

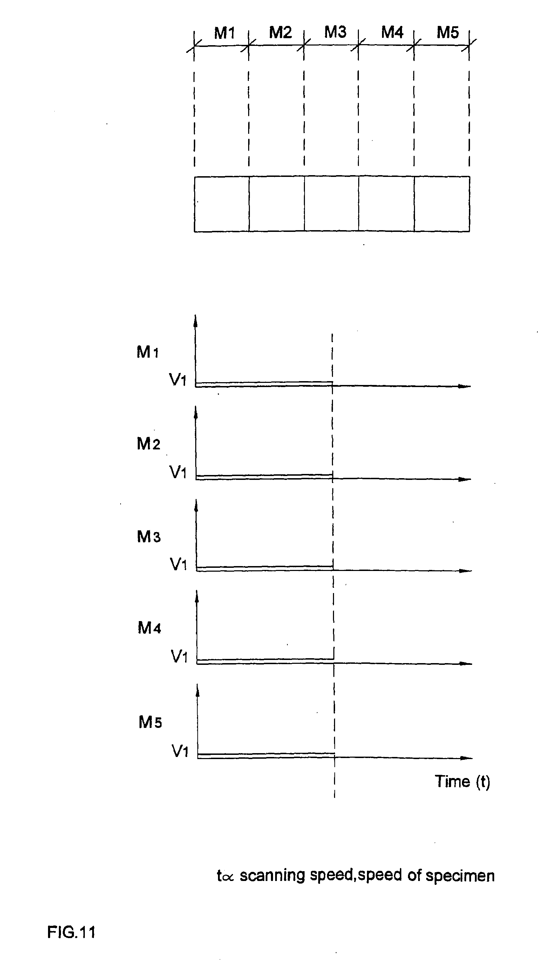

The response of the array of detectors to the hairs in their various orientation positions and core body of the elongated material is explained through Figures 11-14.

**Figure 11** shows the response of the array of detectors in measuring zones when there is no test material between the light source 111 and detectors 113. An array of detectors can have "n" number of detectors depending on the configuration preferred. However for the easy understanding, 5 measurement zones M1 to M5, each denoting to a detector, are shown in Fig. 11.

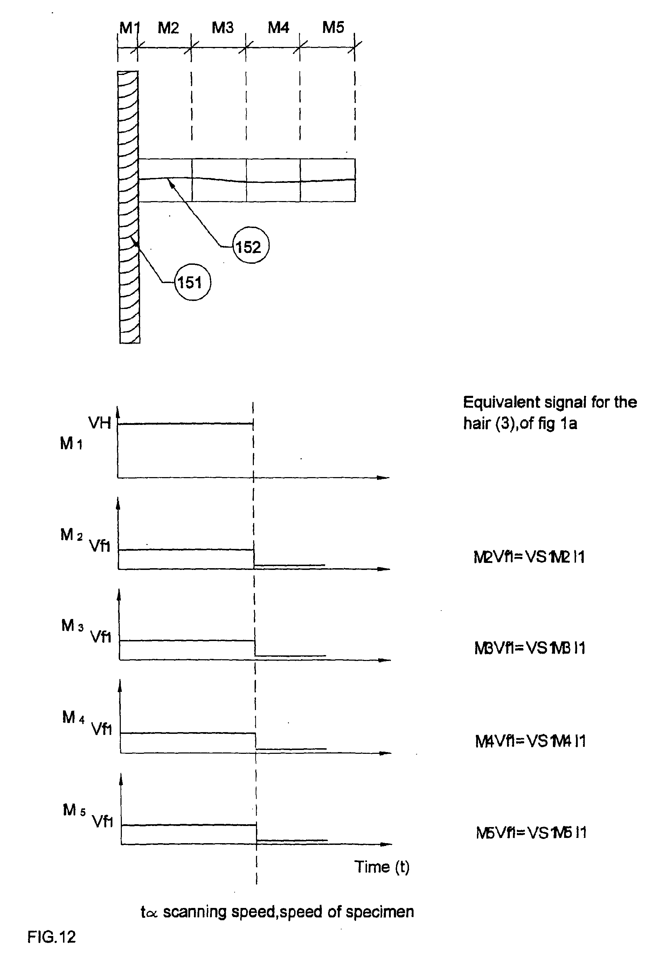

**Figure 12** shows the response of the array of detectors in measuring zones when a portion of an elongated material 151 with a single hair 152 placed in perpendicular to the axis of elongated material is placed between the light source 111 and detectors 113. Arrays denoted by M2 to M5, in Figure 12 show the response of the hair in the said description.

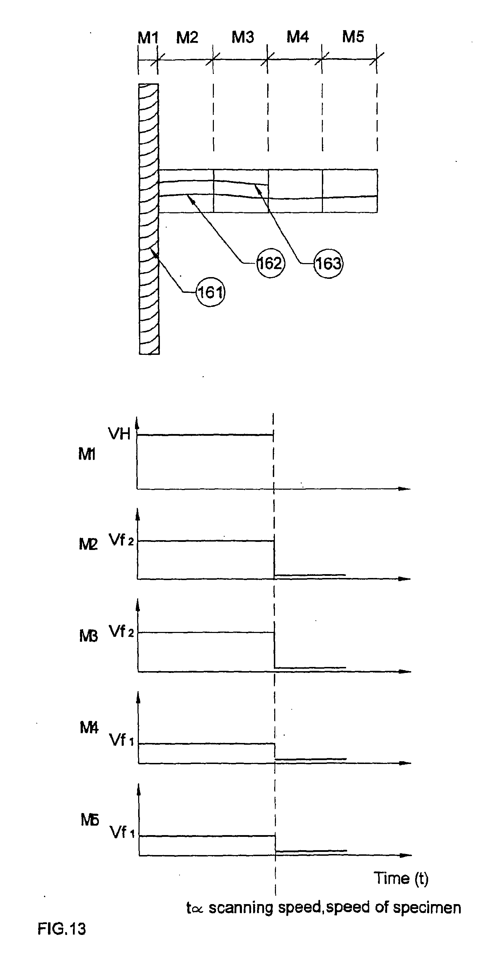

**Figure 13** shows the response of the array of detectors in measuring zones when a portion of an elongated material 161 with two single hairs 162 and 163 present in single scan, each with a different hair length, placed in perpendicular to the axis of elongated material, is placed between the light source 111 and detectors 113. Arrays M2 and M3 return signals equivalent to 2 hairs whereas arrays M4 and M5 return signals correspond to the portion of the longer hair 163.

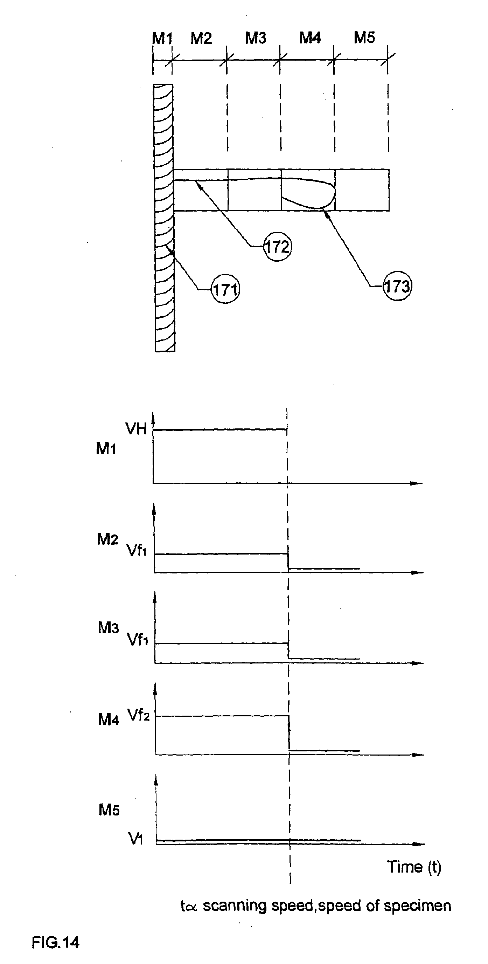

**Figure 14** shows the response of the array of detectors in measuring zones when a portion of an elongated material 171 with a hair 172 having a hooked pattern at the edge 173 within a single scan, is placed between the light source 111 and detectors 113.

Through Figs. 11 to 14, signal $V_H$ is used to denote the response of the detector when the elongated material blocks entire beam and detector receives no portion of the light beam. Such a case is observed with Measuring Zone M1 in Figures 11 to 14, where the portion or full of the core body blocks the measuring zone M1.

Signal $V_1$ is used to denote the response of the detector when no test material is present between light source and detectors. Example of this situation is available in the measurement zone M5 of Figure 14.

Signal $Vf_1$ is used to denote the response of the detector for a single hair. Examples of this are available in Figure 12 corresponding to the measurement zones M2 through M5 and also in Figure 13 through M4 and M5.

Signal $Vf_2$ is used to denote the response of the detector when two independent hairs are projected over the measurement zone. Examples of this are available in Figure 13 corresponding to the measurement zones M2 and M3 and also in Figure 14 in M4 to represent the hooked fibre portion.

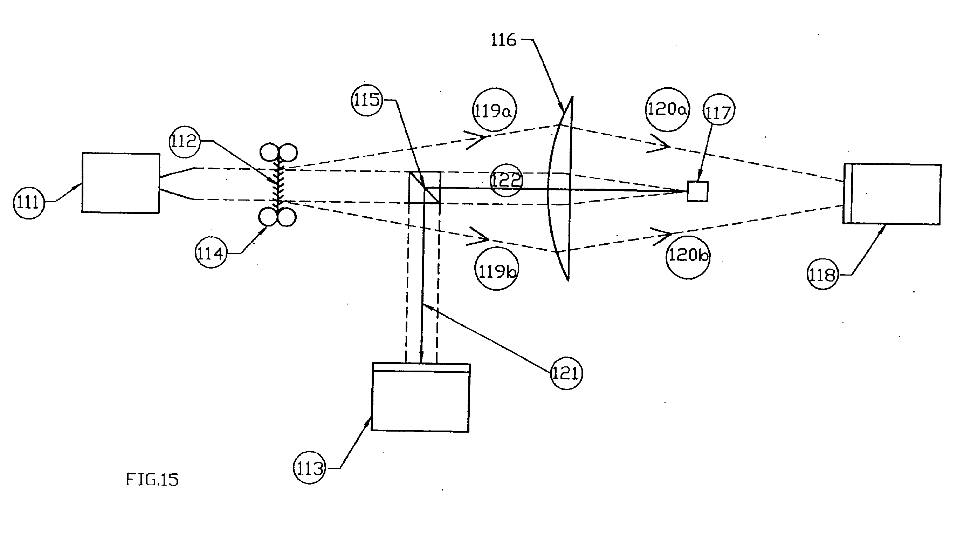

A further preferred device is referred in **Figure 15**. The embodiment comprises a light source 111, preferably for producing a coherent linear beam, a beam splitter 115, an array of detectors 113 including CCD elements displaced at a pre - defined positions, a collecting lens 116 a mechanical filter 117 and another detector 118.

The elongated material 112, preferably the textile spun yarn for which the yarn hairiness is to be estimated is positioned to run between the light source 111 and the beam splitter 115 using pairs of roller 114 running at a constant pre -selected speed. The beam splitter receives light from the source and splits the light into two different axes without deforming the shape of the beam. The first detector, array of detectors 113, is positioned at an angle, preferably at 90 degree to the light source. The second detector, detector 118, is placed along the axis of the light source.

When an elongated material 112 is moved between the light source and the detectors, the core body 112a and the projected hairs 112b of the elongated material respond in the following discussed manner.

The core body 112a and the projected hairs 112b of the elongated material block the light passing through. The beam splitter then receives only the lights that have escaped through and travels in the desired path. The beam splitter 115 splits the light information into two axes 121 and 122. Along the first axis 121, the first detector 113 is placed. The mechanical filter 117 is placed along the second axis 122. The quantum of the light received at the first detector 113 is in proportion to the thickness of the core body and the protruding hairs. The purpose of the mechanical filter is to block light travelling along the second axis 122 so that this light, which represents the core body of the elongated material, does not reach the second detector 118.

The projecting hairs 112b scatter /reflect the light falling over them. The scattered /reflected light then passes through the paths between 119a and 119b and reaches the collecting lens 116. The converged light from the collecting lens 116 then passes through the paths between 120a and 120 b and gets collected by the second detector, detector 118. The total quantum of light thus received at the second detector is a measure of the total hairs passed through the light source.

Next, another method for determining yam properties based on hairs and using a device such as the one according to the above embodiments, is shown.

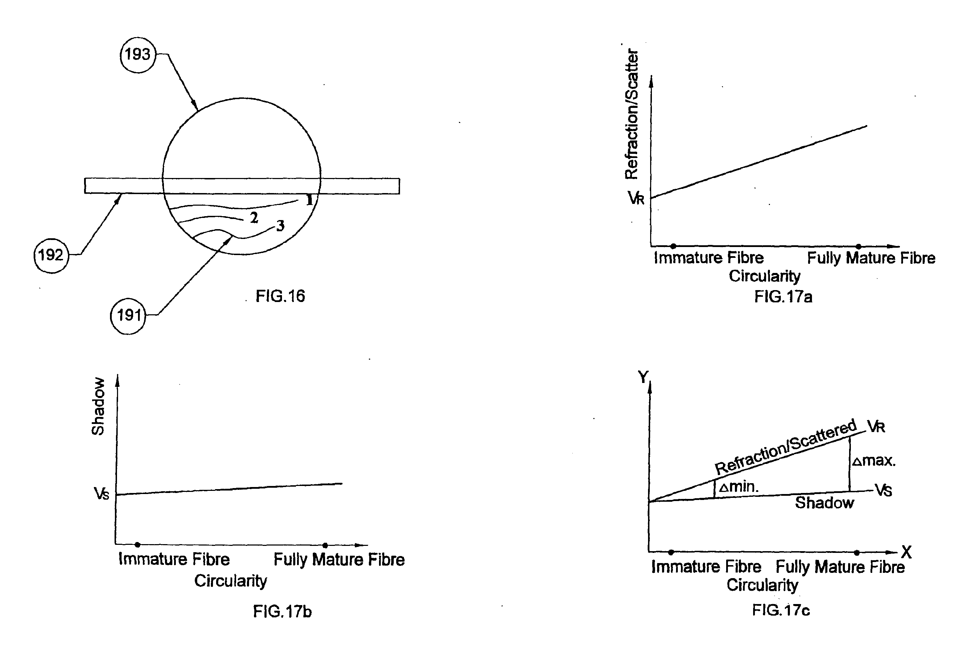

In **Figure 16**, the arrangement of a few hairs 191 in the measuring zone is shown. The shadow measurement is effected using a slit 192; the slit can be of any variable dimension. The reflected/scattered light from the hairs 191 from the light source 193 is collected at the second detector 118. This is used to quantify the reflected/scattered signal of the hairs.

**Figure 17A** shows the relation between the circularity of the fibre, typically cotton, and the reflected / scattered signal $V_R$. As is explained, as the circularity changes to matured stage from immature stage, the reflected / scattered signal increases in a proportion.

**Figure 17B** explains the relation between the circularity of the fibre, typically cotton, and the shadow signal $V_s$ as collected by a Detector 113 of Figure 15. As can be seen, the shadow signal of immature fibre is less compared to the shadow signal of mature fibre. However, the proportion of the change in shadow signal for these mature and immature fibres is less when pitched against the corresponding change in reflection / /scatter signal $V_R$, as can be seen from **Figure 17C.**

Assuming the difference in shadow and reflection / scatter signal is $\Delta$, then the signal difference for an immature fibre will be $\Delta$ min and mature fibre will be $\Delta$ max. The relation between $\Delta$ min and $\Delta$ max is expected to be

$$\Delta \min < \Delta \max \ (7)$$

Using the behaviour as explained in the equation (7), a relationship between shadow signal and reflected /scattered signal could be established in any specific manner. The established relation can be used for process optimisation etc.

One such example demonstrated here uses the ratio of reflected / scatter signal ($V_R$) to the shadow signal ($V_s$) for the same portion of hairs.

$$\text{Ratio} = V_R / V_S. \ (8)$$

The Ratio value estimated for an elongated textile material, say for an example cotton spun yarn, can be used as for optimising processes such as dyeing and mercerising.

**Claims**

1. Device for determining optical characteristics of a thread-like element, especially being a module of an apparatus for simultaneously testing or measuring a yarn and a sliver and/or roving material or a module of an on-line textile environment, the device comprising
   a non-monochromatic light source (52),
   a light frequency sensitive light detector (55),
   a path for the thread-like element (24a), and transport means for moving the thread-like element with constant speed along the path,
   the light source (52) and the detector (55) being placed in a manner that the thread-like element is illuminated by light emitted from the light source and that a proportion of light emitted from the thread-like element upon illumination by the light source is directed to the detector,
   the device further comprising signal processing means (56) which are communicatively coupled to the detector and are programmed in a manner that they can carry out the following steps:

   - from a time dependent signal of the detector (55), evaluating a time dependent colour signal indicative of the colour of thread-like element passing along the path,
   - from the time dependent colour signal, evaluating a time dependent colour deviation information indicative of deviations of the colour from the average colour of the thread-like element,
   - correlating said time dependent colour deviation information with a time dependent quantitative signal deviation information, and
   - determining, based on information whether the time dependent colour deviation information and the time dependent total signal deviation are correlated, fault information indicative of the nature of faults of the thread-like element.

2. The device according to claim 1, wherein the processing means (56) are such that a time dependent quantitative signal is evaluated as a sum of the signals obtained for different light frequencies in the detector.

3. The device according to claim 1 or 2 comprising a second light source (51) and a second detector (54), placed in a manner that, upon illumination of the thread-like element by the second light, it receives a light signal indicative of the thread-like element thickness, this signal being a time dependent quantitative signal, the total signal deviation information being evaluated as a deviation from the average of the quantitative signal for obtaining a fault signal.

4. A method for determining optical characteristics of a thread-like element, , comprising the steps of

   - moving the thread-like element along a path with constant speed,
   - illuminating the thread-like element with non-monochromatic light,
   - detecting light emitted by the thread-like element in a light sensitive manner, as a function of time to obtain a time dependent detector signal,
   - from the time dependent signal, evaluating a time dependent colour signal indicative of the colour of thread-like element passing along the path,
   - from the time dependent colour signal, evaluating a time dependent colour deviation information indicative of deviations of the colour from the average colour of the thread-like element,
   - correlating said time dependent colour deviation information with a time dependent quantitative signal deviation information, and
   - determining, based on information whether the time dependent colour deviation information and the time dependent total signal deviation are correlated, fault information indicative of the nature of faults of the thread-like element.

5. The method according to claim 4, wherein the time-dependent colour signal and the time-dependent total quantitative signal deviation information are detected each using a specific detector (55, 54).

# Fig.1a

Fig.1b

EP 1 624 302 A2

Fig.2A

Fig.2B

Fig.2C

Fig.2D

Fig.2

15

Fig.3

Fig.3A

Fig.3B

Fig.3C

EP 1 624 302 A2

24a

51

54

56

55

52

Fig.4

Fig.5

Fig.6

MASS MEASUREMENT(example Yarn)

SIGNAL PROCESSOR & CONTROLLER

(YS) CAPACITANCE SENSOR

PROCESSOR1 CSP

PROCESSOR2 SPG

PROCESSOR3 IPI

REPORTS

U%, CV%
CV%(CUT LENGTH)
DIAGRAM
VL CURVES
HISTOGRAM

SPECTOGRAMS

THIN PLACE
THICK PLACE
NEPS

CALCULATION / I/P CONTROLLER CIOC 1

COM1

MOXA CARD

PC

Fig.7

EP 1 624 302 A2

FIG.8A

FIG.8B

FIG.8C

M - Measurement zone
S - Scan
L - Length of Hair

FIG.9

Fig.10

tα scanning speed,speed of specimen

FIG.11

M1   M2   M3   M4   M5

(151)

(152)

VH
M 1

Equivalent signal for the
hair (3),of fig 1a

M 2   Vf1

M2Vf1=VS1M2 I1

M 3   Vf1

M3Vf1=VS1M3 I1

M 4   Vf1

M4Vf1=VS1M4 I1

M 5   Vf1

M5Vf1=VS1M5 I1

Time (t)

t∝ scanning speed,speed of specimen

FIG.12

M1   M2   M3   M4   M5

161

162  163

VH

M1

M2   Vf₂

M3   Vf₂

M4   Vf₁

M5   Vf₁

Time (t)

t∝ scanning speed, speed of specimen

FIG.13

FIG.14

FIG.15

EP 1 624 302 A2

Immature Fibre   Fully Mature Fibre
Circularity
FIG.17a

Refraction/Scatter   $V_R$

Refraction/Scattered   $V_R$
Δmax.
Δmin.
$V_S$
Shadow
Immature Fibre   Fully Mature Fibre
Circularity
FIG.17c

FIG.16

193
192
191

Immature Fibre   Fully Mature Fibre
Circularity
FIG.17b

Shadow
$V_S$